# EUROPEAN PATENT APPLICATION

(11) **EP 3 115 342 A1**
(43) Date of publication of application: **11.01.2017**
(21) Application number: 16174204.4
(22) Date of filing: 13.06.2016
(51) Int. Cl.: C02F 3/28, C02F 1/40, B01D 21/00, B03B 5/40, C02F 1/00

(54) **PLANT AND METHOD TO TREAT WASTE**

(30) Priority: 12.06.2015 IT UB20151054
(71) Applicant: N.R.E. Research S.r.l., 34149 Trieste (IT)
(72) Inventor: Nider, Pietro, 34100 Trieste (IT)
(74) Representative: Petraz, Gilberto Luigi

(57) **Abstract**

A plant for treating waste comprising a treatment tank (12) configured to contain the waste and a separation liquid to separate, by floating, in the waste, parts of inorganic material from parts of organic material. The plant comprises an apparatus for anaerobic digestion (14) and a delivery circuit (13) that connects the treatment tank (12) and the anaerobic digestion apparatus (14) in order to transfer the separation liquid and the organic material from the treatment tank (12) to the anaerobic digestion apparatus (14), configured to treat the organic material separated in the treatment tank (12) and to produce combustible gas and biomass.

## Description

### FIELD OF THE INVENTION

The present invention concerns a plant and a method to treat waste, for example to select and/or homogenize it.

In particular, the present invention can be used to treat for example organic waste deriving from differentiated domestic collection, or from non-domestic users such as for example bars, restaurants, canteens or suchlike.

### BACKGROUND OF THE INVENTION

The growing quantities of urban solid waste (USW) is one of the biggest ecological problems, for which different types of plant have been designed to dispose of it.

For example, it is known to use waste-to-energy plants and pyrolysis or massification plants to dispose of urban solid waste, but these are only partly successful.

One disadvantage of known solutions is that they are very expensive, both in economic terms and also in terms of environmental impact. Indeed, these technologies are based on processes where the waste is treated hot and produce residues, often special residues, which require disposal, generally in landfills, and can also generate powders and dangerous emissions for the environment.

To try to overcome the problem of waste disposal, the practice of differentiated waste collection is increasingly common, to recover and recycle most of the urban solid waste, such as plastic, glass, iron, aluminum. These materials have some value on the market and can generate income, which can be used to pay the costs of disposing of humid or organic waste, which needs suitable plants to be treated and disposed of.

MBT (Mechanical-Biological Treatment) type plants are also known, which perform a cold treatment to separate the organic fraction and the recyclable materials, allowing a reduced use of landfills and incinerators.

The separation of the waste into organic and inorganic allows to exploit the OFUSW (Organic Fraction of the Urban Solid Waste) to produce energy and compostable material.

Plants are known, which separate the organic material in the urban solid waste (USW) produced by domestic and/or non-domestic users and treat it using an anaerobic digester, to produce biogas and compost.

In known plants, the waste is generally fed on a conveyor belt, and the plastic, paper and glass is gradually recovered by operators positioned on the sides of the conveyor belt, or by suitable mechanical arms.

One disadvantage of these plants is that the organic material is not completely separated from the inorganic material, and therefore the organic material obtained by the separation process is not homogeneous and contains residues of inert materials and/or plastic.

One disadvantage of known plants is that an organic material is fed into the anaerobic digesters that is not homogeneous enough, and therefore the anaerobic digestion process takes a long time; moreover, the digesters need a lot of maintenance, with a consequent at least partial stoppage of the plant. The residues of inorganic material, in fact, tend to sediment in the digester, obstructing the pipes, so that it is necessary to stop the treatment plant frequently in order to clean them.

Another disadvantage is that the use of non-homogeneous organic material with residues of inorganic material leads to a low-quality biomass and compost, so using these is not best for the environment.

A treatment plant for urban solid waste is also known that comprises a treatment tank containing a separation liquid, generally water, into which the urban solid waste is introduced on each occasion. Thanks to the fact that the types of urban solid waste float in different ways, the separation liquid allows to separate them from each other. A separation device provides to partly separate one type of waste from another. However, this solution is particularly complex because it requires a treatment to recover and purify the separation liquid. This solution has extremely high management costs and is usually used for large-size plants since the costs are not justified for smaller plant sizes.

One purpose of the present invention is to obtain a plant for treating waste which allows to obtain high-quality biogas, compost and/or biomass.

Another purpose of the present invention is to obtain a plant for treating organic waste which is environmentally friendly, that is, which does not produce dangerous emissions for the environment.

Another purpose of the present invention is to supply a plant for treating waste which does not require frequent maintenance and which can therefore work in a more productive and efficient way than known plants.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes, a plant for treating urban solid waste comprises a treatment tank configured to contain said waste and a separation liquid to carry out a process of separation, for example of the gravitational hydrodynamic type, of the waste and separate by floating, at least parts of inorganic material from parts of organic material.

According to the present invention, the treatment plant comprises an apparatus for anaerobic digestion and a delivery circuit that connects the treatment tank and the anaerobic digestion apparatus in order to transfer the separation liquid and the organic material from the treatment tank to the anaerobic digestion apparatus. The anaerobic digestion apparatus is configured to treat the organic material separated in the treatment tank and to produce combustible gas and biomass.

Moreover, according to another aspect of the invention, the plant comprises a recirculation circuit, connected to the anaerobic digestion apparatus and to the treatment tank, in order to recirculate the separation liquid from the anaerobic digestion apparatus toward the treatment tank.

This allows to recirculate continuously a large part of the separation liquid between the separation tank and the anaerobic digestion apparatus, preventing useless waste. Following the digestion processes that occur automatically inside the anaerobic digestion apparatus, the organic material is removed from the separation liquid which can be recirculated toward the separation tank without requiring further treatment/purification apparatuses as used in the known separation plants described above.

The amount of biomass or compost obtained at the end of the anaerobic digestion process is generally equal to about 10% of the overall organic material supplied on entrance, while about 90% of the material fed to the anaerobic digestion apparatus consists of liquid, substantially water.

The present invention also concerns a method to treat waste, which provides to feed waste into a treatment tank filled with a separation liquid to separate in the waste, by floating, parts of inorganic material from parts of organic material.

According to one aspect of the present invention, the method provides to transfer the separation liquid and the organic material from the treatment tank toward an anaerobic digestion apparatus, to treat the organic material separated in the treatment tank and produce combustible gas and biomass, and to recirculate the separation liquid from the anaerobic digestion apparatus to the treatment tank.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a schematic view of a plant to treat organic waste in accordance with a possible embodiment;
- fig. 2 is a schematic view of a treatment tank installable in the plant in fig. 1.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

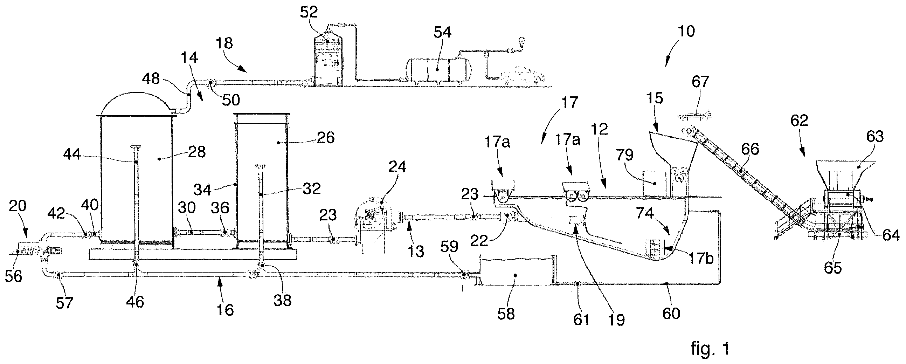

Embodiments described here using fig. 1 concern a plant 10 for treating waste which comprises a treatment tank 12 configured to contain waste, and a separation liquid.

The separation liquid allows to perform a separation process, by floating, of parts of inorganic material from parts of organic material.

The different floating modes of the types of waste, or specific weight, allow to separate the various types of material.

The separation liquid used can be water, for example.

In particular, the waste that has a specific weight greater than the specific weight of the separation liquid tends to precipitate onto the bottom of the treatment tank 12, while the waste with a specific weight lower than that of the separation liquid tends to float on the surface of the separation liquid. The organic material that has a specific weight substantially equal to that of the separation liquid remains substantially suspended in the latter.

Generally speaking, the inert materials, such as ferrous metals, sand, and stone, tend to deposit on the bottom of the treatment tank 12, while the light materials, such as plastic materials, tend to float on the top of the separation liquid, and the organic and biodegradable material tends to remain suspended, because it has a specific weight substantially equal to that of the separation liquid.

Thanks to the separation in water, it is possible to obtain very high recovery percentages of inorganic waste compared with other separation methods. Moreover, by pouring the waste into water, a considerable reduction in the emission of noxious powders and bad smells is obtained.

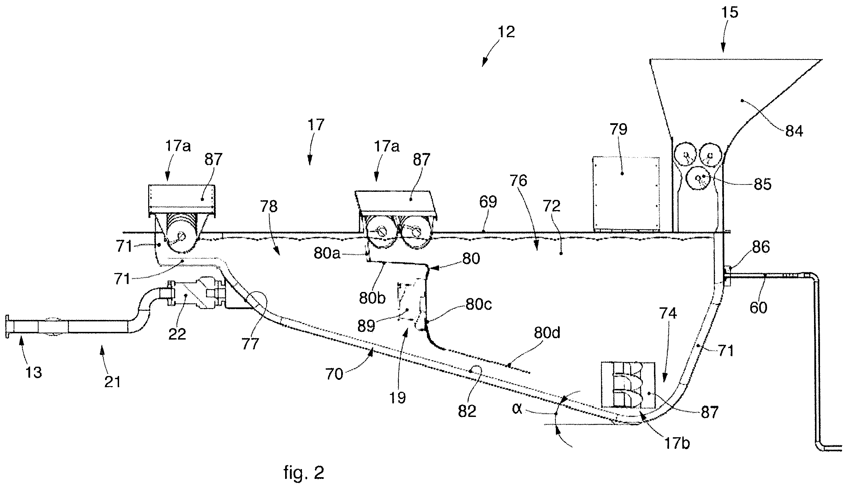

According to the solution described in figs. 1 and 2, the treatment tank 12 is provided with feed means 15, configured to introduce the waste to be treated into the treatment tank 12.

According to embodiments described using fig. 2, the feed means 15 comprise a loading hopper 84, configured to receive the waste of organic and inorganic material, for example from a feed unit 62, and to supply it to the treatment tank 12.

The feed unit 62, as shown in fig. 1, is disposed upstream of the feed means 15 and the waste to be treated is conferred therein.

According to the solution shown in fig. 1, the feed unit 62 can comprise a shredder device 64, configured to at least partly shred the waste and possibly, if it is collected in plastic bags, to break the latter.

On the bottom of the shredder device 64 a suction device 65 can be provided, configured to suck in the broken plastic bags and to dispose of them directly.

According to some embodiments, the feed unit 62 also comprises a conveyor belt 66 configured to transport the waste, partly shredded by the shredder device 64, to the feed means 15 of the treatment tank 12.

According to possible solutions described using fig. 1, the feed unit 62 can comprise a magnetic device 67, for example positioned above the conveyor belt 66 and configured to attract the ferromagnetic materials present in the waste and to remove them before they are introduced to the feed means 15. The magnetic device 67 can comprise a permanent magnet, or a selectively activated electromagnet.

The feed means 15, according to the solution shown in fig. 2, can comprise a crushing device 85, located in the loading hopper 84 and configured to crush the waste contained therein.

The treatment tank 12 is open toward the outside near an end edge 69 in correspondence with which the waste is introduced.

The treatment tank 12 is defined by at least one bottom wall 70 and by lateral walls 71 defining with the bottom wall 70 a containing compartment 72 for the separation liquid.

According to a possible solution, the containing compartment 72 has a collection zone 74 with a concave conformation in which the inorganic materials that precipitate onto the bottom can be collected.

According to a possible solution, the bottom wall 70 is inclined with respect to the horizontal, that is, with respect to the end edge 69, by an angle α with an amplitude comprised between about 10° and about 30°, for example about 20° with respect to the horizontal.

In particular, it is provided that the bottom wall 70 is inclined downward in the direction of the collection zone 74.

The angle of inclination allows to convey, through gravity, the inorganic material that precipitates onto the bottom wall 70 toward the collection zone 74.

According to possible solutions, not shown in the drawings, the treatment tank 12 can be provided with a plurality of collection zones 74, defined for example by a plurality of inclined bottom walls 70.

According to the solution shown in figs. 1 and 2, a dividing wall 80 can be associated with the treatment tank 12, configured to divide the treatment tank 12 into a first sector 76 and a second sector 78, in both of which the organic and inorganic materials of the waste are separated through floating.

The dividing wall 80 can extend from the end edge 69 of the treatment tank 12 toward the inside, and for part of the depth thereof.

According to some embodiments, the bottom wall 70 extends between the first sector 76 and the second sector 78.

According to this solution, the collection zone 74 can be provided in the first sector 76.

The first sector 76 can be associated with the feed means 15 of the waste, and in it occurs the first insertion of the waste and a first separation through floating of the organic material from the inorganic material.

According to the solution shown in figs. 1 and 2, the first sector 76 and the second sector 78 are in fluidic connection with each other by means of a passage channel 82 which allows the separation liquid to pass, and possibly also part of the waste, from the second sector 78 to the first sector 76, or possibly, according to one solution not shown, vice versa.

The passage channel 82 can be defined between the dividing wall 80 and the bottom wall 70 of the treatment tank 12.

In this way, thanks to the inclination of the bottom wall 70, the inorganic materials which are deposited on the portion of the bottom wall 70 comprised in the second sector 78 are transferred from the second sector 78 to the first sector 76 passing through the passage channel 82 to deposit in the collection zone 74.

According to the solution shown in fig. 2, the dividing wall 80 can comprise four segments 80a, 80b, 80c, 80d, disposed angularly offset with respect to each other, which in section define a stepped conformation. The first segment 80a is positioned vertical, the second segment 80b is positioned substantially orthogonal to the first segment 80a, in a horizontal direction, possibly slightly inclined downward, the third segment 80c is positioned vertical and the fourth segment 80d is substantially parallel to the bottom wall 70 and defines with the latter the passage channel 82.

It is quite evident that the treatment tank 12 according to the present invention can comprise more than one dividing wall 80 to divide the containing compartment 72 into more than two sectors, in each of which separation of the waste can take place in successive steps, through floating.

According to possible solutions of the present invention, the dividing wall 80 can be associated with movement devices, not shown in the drawings and configured to move the dividing wall 80 in the treatment tank 12.

According to a possible embodiment of the present invention, the treatment tank 12 can comprise removal means 17 configured to remove from the treatment tank 12 the inorganic materials that have separated from the organic materials.

According to possible solutions, see figs. 1 and 2 for example, the removal means 17 can comprise first removal members 17a associated with the upper part of the containing compartment 72 and configured to remove the inorganic materials floating on the surface of the separation liquid, and second removal members 17b associated with the bottom of the containing compartment 72 and configured to remove the inorganic materials that have precipitated onto the bottom of the containing compartment 72.

According to a possible solution, the first removal members 17a and the second removal members 17b comprise an extractor device 87 chosen from a group comprising a double-screw extractor or a single-screw extractor.

According to the solution shown in fig. 2, the first removal members 17a are mobile above the treatment tank 12, to remove the inorganic materials floating on the separation liquid. Merely by way of example, the first removal means 17a are mobile parallel to and on the end edge 69 of the treatment tank 12.

According to the solutions in which there are one or more dividing walls 80, respective first removal members 17a can be associated with each sector defined by said dividing wall/walls 80.

For example with reference to fig. 2, two first removal members 17a are provided, of which one is associated with the first sector 76 and one with the second sector 78.

The first removal members 17a associated with the first sector 76 are installed in correspondence with the dividing wall 80 to collect more easily the waste that accumulates in the portion of the treatment tank 12 delimited by the dividing wall 80.

According to the solution shown in figs. 1 and 2, the second removal members 17b are associated with the collection zone 74 to remove the inorganic materials that are conveyed in the latter.

According to a possible solution, between the first sector 76 and the second sector 78 transfer means 19 are interposed, configured to transfer the separation liquid and the waste contained in the first sector 76 to the second sector 78.

According to the solution shown in figs. 1 and 2, the transfer means 19 can be attached to the dividing wall 80 to transfer the separation liquid and the waste contained in the first sector 76 to the second sector 78.

According to a possible solution, the transfer means 19 can be installed in an intermediate position of the height of the dividing wall 80, that is, about half way up the treatment tank 12. In this way, the transfer means 19 are configured to transfer the organic material remaining suspended in the separation liquid, and not the part of material that has separated through precipitation or floating.

The transfer means 19 can comprise, for example, a pumping device to pump the separation liquid from the first sector 76 to the second sector 78.

The transfer means 19 therefore allow to perform in the second sector 78 a further action of separating the organic and inorganic material, if the latter has not been already separated.

According to a possible solution, the transfer means 19 can also comprise a shredder device 89, configured to shred the organic material into particles with sizes less than 3 mm. The shredding possibly allows to free possible parts of organic material that are trapped in the inorganic material, thus obtaining an optimized separation.

The inorganic material floating on the separation liquid is removed by the first removal members 17a installed in the second sector 78 while the inorganic materials that precipitate on the bottom wall 70 due to the inclination thereof are transferred to the collection zone 74 where they are removed by the second removal members 17b.

According to the solution shown in figs. 1 and 2, the treatment tank 12 is provided with an introduction aperture 86, configured to allow the introduction of the separation liquid into the containing compartment 72, and a discharge aperture 77. According to a variant embodiment, not shown in the drawings, the separation liquid can be introduced and discharged by introduction pipes and discharge pipes, inserted in the treatment tank 12.

The discharge aperture 77 is made in an intermediate position of the height of the second sector 78 of the treatment tank 12, thus allowing to discharge the organic material and the separation liquid and not the inorganic materials which have separated due to floating or precipitation.

According to possible solutions, the treatment tank 12 can comprise stirring means 79, positioned in correspondence with the upper edge of the treatment tank 12 and configured to move the light elements that rise to the surface and to direct them to the first removal members 17a. According to some embodiments, the stirring means 79 can comprise nozzles to deliver jets of separation liquid at a pressure comprised between 3atm and 5atm.

According to a possible solution shown in figs. 1 and 2, the plant 10 comprises a delivery circuit 13, configured to transfer the separation liquid and the organic material from the treatment tank 12 to an anaerobic digestion apparatus 14.

The delivery circuit 13 can be connected to the discharge aperture 77 described above.

According to some embodiments, the delivery circuit 13 can comprise a shredder pump 22 configured to suck in and shred the separation liquid and the organic material present in the treatment tank 12, and to obtain a compost of organic material as homogeneous and uniform as possible, to be sent to the anaerobic digestion apparatus 14.

According to some embodiments, the shredder pump 22 can be configured for example to reduce the particles of organic material to a size of about 0.5 mm, which allows to have a watery and homogeneous organic substance.

According to some embodiments, downstream of the shredder pump 22, the delivery circuit 13 can comprise a filtering element 24, the function of which is to filter possible residual particles of inorganic material, which are still present after passage in the treatment tank 12.

The filtering element 24 can for example separate possible residues of sand and/or glass from the organic material, supplying at exit the separation liquid and the filtered and separated organic material, ready to be processed in the anaerobic digestion apparatus 14.

According to some embodiments, the filtering element 24 can be a drum-type screen or riddle for example, able to recover elements with sizes above 0.25 mm, so as to supply at entry to the anaerobic digestion apparatus 14 a compost as homogeneous as possible.

Valves 23 can be provided, located upstream and downstream of the filtering element 24 and able to be selectively activated to allow or prevent the flow of organic material in the delivery circuit 13 and through the filtering element 24.

The anaerobic digestion apparatus 14 is configured to treat the organic material separated by the treatment tank 12 and to produce combustible gas and biomass.

According to some embodiments, the anaerobic digestion apparatus 14 comprises one or more containers 26, 28 in which the separation liquid and the organic material are subjected to an anaerobic digestion process to produce biogas.

In the containers 26 and 28 the separation liquid separates from the organic material and is extracted through a recirculation circuit 16 connected between the containers 26 and 28 and the treatment tank 12.

The recirculation circuit 16 allows to return to circulation in the plant 10 the same separation liquid that previously performed the function of separating the waste, and which has become necessary for the digestion processes in the anaerobic digestion apparatus 14.

According to the embodiment shown in fig. 1, the anaerobic digestion apparatus 14 comprises a digester container 26 and a post-digestion container 28, connected by a pipe 30.

According to variant embodiments, a single digester container can be provided to perform the entire anaerobic digestion process, or several digester containers in series with each other.

According to embodiments described using fig. 1, the digester container 26 can be the UASB type (Up-flow Anaerobic Surge Blanket Reactor), in which the steps of hydrolysis and acetogenesis are performed, with very fast retention times, for example comprised between 22 and 48 hours.

During the hydrolysis and acetogenesis steps, the first separation takes place in the digester container 26 of the solid or digested part and the liquid, and gas is possibly generated.

The liquid can be recovered and sent to the recirculation circuit 16. To this purpose, the digester container 26 can comprise a pipe for the liquid to exit 32, connected to the recirculation circuit 16, through which the liquid is discharged.

According to some embodiments, a pump 38 can be connected to the pipe for the liquid to exit 32, to transfer the liquid from the digester container 26 to the recirculation circuit 16.

According to some embodiments, stirring means (not shown) can be provided in the digester container 26, configured to mix the organic material present and keep it moving, and to promote the digestion process.

According to some embodiments, the digester container 26 can also comprise a gas outlet pipe 34 from which the gas generated in the digester container 26 is removed and fed by the pipe 30 to the post-digestion container 28.

A pump 36 is connected to the pipe 30, between the digester container 26 and the post-digestion container 28, with the function of taking in the gas and the digested part present in the digester container 26, and to feed them to the post-digestion container 28.

According to some embodiments, the methanogenesis step takes place in the post-digestion container 28, by means of which a further separation is obtained of the liquid and solid, and gas is generated. To be completed, the methanogenesis step requires about 20-28 days, so that the post-digestion container 28, according to some embodiments, can be sized so that it can work and treat the organic material arriving from the digester container 26 without having to interrupt the process.

A further digestion of the material arriving from the digester container 26 also takes place in the post-digestion container 28 with the separation of the liquid and solid part, and the generation of combustible gas or biogas.

The post-digestion container 28 can have a capacity sufficient to contain the solid part digested in about 20 days. At exit from the post-digestion container 28 a quantity of digested solid part is obtained that can be equal, for example, to about 10% of the total mass of organic material supplied at entry to the anaerobic digestion apparatus 14.

The post-digestion container 28 can comprise a pipe for the liquid to exit 44, connected to the recirculation circuit 16, through which the separated liquid part is discharged.

According to some embodiments, a pump 46 can be connected to the pipe for the liquid to exit 44, to transfer the liquid from the post-digestion container 28 to the recirculation circuit 16.

Thanks to the fact that a homogeneous organic material is used, substantially without any residues of inert and/or plastic materials, both the compost and the biogas produced are of high quality. The biogas produced, for example, can have a concentration of methane (CH₄) between about 70% and about 75% with respect to the total quantity of gas produced.

In the post-digestion container 28 the solid part digested is deposited in the lower part, the gaseous part generated rises upward to occupy the upper part, while the liquid part remains in the central zone where it is discharged by the pipe for the liquid to exit 44.

According to some embodiments, stirring means, not shown, can be provided in the post-digestion container 28, configured to mix the organic material and keep it moving in the lower part of the post-digestion container 28, to promote the digestion process.

According to some embodiments, the post-digestion container 28 can comprise an exit pipe for the digestate 40, connected to biomass extraction means 20.

According to some embodiments, between the exit pipe for the digestate 40 and the biomass extraction means 20 a pump 42 can be provided, able to be selectively activated to suck in the solid part digested and generated in the post-digestion container 28 and to supply it to the biomass extraction means 20.

According to embodiments described using fig. 1, the biomass extraction means 20 can comprise a separation device 56, for example a solid/liquid separator, configured to separate the solid part completely from the liquid part. The liquid part can be made to flow toward the recirculation circuit 16, selectively by means of a valve 57, to be subsequently re-used, while the solid part can be further worked to obtain dry compost, substantially without any pollutant elements. According to some embodiments, in series with the separation device 56 drying, mixing and briquetting stations can be provided, not shown in the drawings, in which the compost can be further worked to obtain a dry biomass with a high calorific power, which can be used as a secondary, or recovered, solid fuel (SRF).

According to some embodiments, the post-digestion container 28 can also comprise a gas exit pipe 48, configured to remove the gas produced in the post-digestion container 28 and to feed it to gas extraction means 18.

According to some embodiments, the gas extraction means 18 comprise a valve 50, connected to the gas exit pipe 48, which can be selectively opened to make the gas produced flow from the post-digestion container 28.

In particular, the valve 50 can be opened to allow the gas to pass when it is mature, that is, when it has reached a determinate concentration of methane, and/or determinate pressure. According to some embodiments, sensors can be provided, not shown in the drawings, to detect the data relating to the methane concentration and/or gas pressure to be subsequently sent to a suitable control unit configured to process the data and command the activation or de-activation of the valve 50 and/or the pumps 36, 38, 42, 46.

According to some embodiments, the gas extraction means 18 comprise a purification apparatus 52, for example a wash tower, in which the gas arriving from the anaerobic digestion apparatus 14 is washed and purified.

According to embodiments described using fig. 1, the gas extraction means 18 can comprise a gas storage device 54, for example a gas cylinder, in which the biomethane gas obtained can be stored.

The biomethane obtained can be used, for example, for auto traction and/or to feed a co-generation plant for the production of electric and thermal energy.

According to embodiments described using fig. 1, the recirculation circuit 16 can comprise a collection tank 58 for the liquid arriving from the anaerobic digestion apparatus 14. Advantageously, the collection tank 58 can be open at the top so that any possible ammonia present in the liquid will evaporate.

The recirculation circuit 16 can also comprise a feed pipe 60 which connects the collection tank 58 to the treatment tank 12.

Upstream of the collection tank 58 a valve 59 can be provided, which can be selectively opened or closed to allow or prevent the passage of the separation liquid to the collection tank 58.

According to some embodiments, between the collection tank 58 and the treatment tank 12, the recirculation circuit 16 comprises a pump 61 configured to transfer the separation liquid from the collection tank 58 to the treatment tank 12.

According to some embodiments, combinable with all the embodiments described here, the plant 10 can comprise a line to suck in and treat the used air, not shown in the drawings, equipped for example with a scrubber and a biofilter to treat and purify the air before it is delivered into the external environment. In this way the plant guarantees both to damp the smells and also the total absence of pollutant emissions.

It is clear that modifications and/or additions of parts may be made to the plant for treating organic waste as described heretofore, without departing from the field and scope of the present invention.

It is also clear that, although the present invention has been described with reference to some specific examples, a person of skill in the art shall certainly be able to achieve many other equivalent forms of plant for treating organic waste, having the characteristics as set forth in the claims and hence all coming within the field of protection defined thereby.

## Claims

1. Plant for treating waste comprising a treatment tank (12) configured to contain said waste and a separation liquid to separate, by floating, in said waste, parts of inorganic material from parts of organic material, **characterized in that** it comprises an apparatus for anaerobic digestion (14) and a delivery circuit (13) that connects said treatment tank (12) and said anaerobic digestion apparatus (14) in order to transfer said separation liquid and said organic material from said treatment tank (12) to said anaerobic digestion apparatus (14), said anaerobic digestion apparatus (14) being configured to treat the organic material separated in the treatment tank (12) and to produce combustible gas and biomass, and **in that** it comprises a recirculation circuit (16), connected to said anaerobic digestion apparatus (14) and to said treatment tank (12), in order to recirculate said separation liquid from said anaerobic digestion apparatus (14) toward said treatment tank (12).

2. Plant as in claim 1, **characterized in that** said delivery circuit (13) comprises a shredding pump (22) to suck in and shred the separation liquid and the organic material present in said treatment tank (12).

3. Plant as in claim 1 o 2, **characterized in that** said delivery circuit (13) comprises a filtering element (24) to filter residual particles of inorganic material.

4. Plant as in any claim hereinbefore, **characterized in that** said recirculation circuit (16) comprises a collection tank (58) to collect the liquid coming from the anaerobic digestion apparatus (14), said collection tank (58) being open at the top to allow the evaporation of the ammonia possibly present in the separation liquid before feeding it to said treatment tank (12).

5. Plant as in claim 4, **characterized in that** said recirculation circuit (16) comprises a pump (61) configured to transfer the separation liquid from said collection tank (58) toward said treatment tank (12).

6. Plant as in any claim hereinbefore, **characterized in that** said anaerobic digestion apparatus (14) comprises at least one container (26, 28) in which the separation liquid and the organic material are subjected to a process of anaerobic digestion and **in that** said container (26, 28) comprises at least one pipe for the liquid to exit (32, 44), connected to said recirculation circuit (16).

7. Plant as in any claim hereinbefore, **characterized in that** said treatment tank (12) comprises removal means (17a, 17b) configured to remove the inorganic materials present in said treatment tank (12).

8. Method for treating organic waste that provides to:
- feed waste into a treatment tank (12) filled with a separation liquid to separate in the waste, by floating, parts of inorganic material from parts of organic material, **characterized in that** it comprises:
- transferring said separation liquid and said organic material from said treatment tank (12) toward an anaerobic digestion apparatus (14),
- treating in said anaerobic digestion apparatus (14) the organic material separated in the treatment tank (12) and producing combustible gas and biomass, and
- recirculating the separation liquid from the anaerobic digestion apparatus (14) to the treatment tank (12).

9. Method as in claim 8, **characterized in that** it comprises shredding the organic material that is transferred from said treatment tank (12) to said anaerobic digestion apparatus (14).

10. Method as in claim 8 or 9, **characterized in that** it comprises filtering inorganic materials possibly present in said separation liquid and said organic material during the transfer of the latter two toward the anaerobic digestion apparatus (14).
